**Europäisches Patentamt**

**European Patent Office**

**Office européen des brevets**

(11) Veröffentlichungsnummer: **0 305 550**
**A1**

# (12) EUROPÄISCHE PATENTANMELDUNG

Veröffentlicht nach Art. 158 Abs. 3 EPÜ

(21) Anmeldenummer: **88903467.4**

(22) Anmeldetag: **25.02.88**

Daten der zugrundeliegenden internationalen Anmeldung:

(86) Internationale Anmeldenummer:
**PCT/SU88/00045**

(87) Internationale Veröffentlichungsnummer:
**WO88/06906 (22.09.88 88/21)**

(51) Int. Cl.³: **A 61 N 1/34**
**A 61 N 1/08**
**//A61C19/08**

(30) Priorität: **17.03.87 SU 4211982**

(43) Veröffentlichungstag der Anmeldung:
**08.03.89 Patentblatt 89/10**

(84) Benannte Vertragsstaaten:
**AT CH DE FR GB IT LI NL SE**

(71) Anmelder: **BUDYKO, Viktor Alexandrovich**
**ul. Mira, 20-60**
**Zaporozhie, 330033(SU)**

(71) Anmelder: **KONOVALENKO, Vladimir Vladimirovich**
**ul. Vodonapornaya, 16a**
**Zaporozhie, 330063(SU)**

(71) Anmelder: **IVANCHENKO, Andrei Fedoseevich**
**ul. Angolenko 14a-17**
**Zaporozhie, 330063(SU)**

(71) Anmelder: **KUTSOV, Valentin Dmitrievich**
**ul. Kedrovaya, 67**
**Zaporozhie, 330077(SU)**

(71) Anmelder: **LASTOCHKIN, Boris Nikolaevich**
**ul. Lenina, 58-4**
**Zaporozhie, 330063(SU)**

(71) Anmelder: **KROKHMAL, Vladimir Mikhailovick**
**ul. Lenina, 58-15**
**Zaporozhie, 330063(SU)**

(71) Anmelder: **ZHDAN, Nikolai Nikolaevich**
**ul. Sytova, 2-32**
**Zaporozhie, 330058(SU)**

(72) Erfinder: **BUDYKO, Viktor Alexandrovich**
**ul. Mira, 20-60**
**Zaporozhie, 330033(SU)**

(72) Erfinder: **KONOVALENKO, Vladimir Vladimirovich**
**ul. Vodonapornaya, 16a**
**Zaporozhie, 330063(SU)**

(72) Erfinder: **IVANCHENKO, Andrei Fedoseevich**
**ul. Angolenko 14a-17**
**Zaporozhie, 330063(SU)**

(72) Erfinder: **KUTSOV, Valentin Dmitrievich**
**ul. Kedrovaya, 67**
**Zaporozhie, 330077(SU)**

(54) **ELEKTROANALGESIE-VORRICHTUNG FÜR GEWEBE VON KRANKEN.**

(57) Die Vorrichtung zur elektrischen Analgesie von Patientengeweben enthält in Reihe geschaltet einen Generator (1) für unsymmetrische Impulse, an dessen Ausgang die einen Impulse eine größere Breite haben, um die elektrische Analgesie der Patientengewebe zu gewährleisten, während die anderen eine geringere Breite zur Gewährleistung der Depolarisation der Patientengewebe haben, einen Stromregler (2) und einen Stromstabilisator (3), an welchen Elektroden (4, 5) zum Anschließen unmittelbar an den Patienten und an ein Mittel zur Einwirkung auf den Patienten angeschlossen sind. An eine der Elektroden (4, 5) ist eingangsseitig eine Einheit (6) zur Erzeugung eines Signals beim Schließen des elektrischen Kreises der Elektroden über die Patientengewebe angeschlossen, wobei der Ausgang dieser Einheit an den Eingang einer Einheit (7) zum Auslösen des Generators für unsymmetrische Impulse ab Impuls der geringeren Breite angeschlossen ist, die ausgangsseitig an den Eingang des Generators (1) für unsymmetrische Impulse angeschlossen ist.

EP 0 305 550 A1

./...

Croydon Printing Company Ltd.

(72) Erfinder: **LASTOCHKIN, Boris Nikolaevich**
**ul. Lenina, 58-4**
**Zaporozhie, 330063(SU)**

(72) Erfinder: **KROKHMAL, Vladimir Mikhailovick**
**ul. Lenina, 58-15**
**Zaporozhie, 330063(SU)**

(72) Erfinder: **ZHDAN, Nikolai Nikolaevich**
**ul. Sytova, 2-32**
**Zaporozhie, 330058(SU)**

(74) Vertreter: **Patentanwälte Beetz sen. - Beetz jun. Timpe -**
**Siegfried - Schmitt-Fumian- Mayr**
**Steinsdorfstrasse 10**
**D-8000 München 22(DE)**

# VORRICHTUNG ZUR ELEKTRISCHEN ANALGESIE 0305550
## VON PATIENTENGEWEBEN

### Technisches Gebiet

Die Erfindung bezieht sich auf die Medizintechnik, insbesondere auf eine Vorrichtung zur elektrischen Analgesie von Patientengeweben.

### Zugrundeliegender Stand der Technik

Zur Zeit existieren sowohl medikamentöse als auch physische Verfahren zur Anästhesie während des Zahnpräparierens. Im Zusammenhang damit, daß die medikamentösen Verfahren bei Patienten allergische Reaktionen hervorrufen können, sind daher besonders perspektivisch die physischen Verfahren, denen zugrunde der Einsatz des elektrischen Gleichstromes liegt, der beim Durchfließen durch die Gewebe eines zu präparierenden Zahns den Effekt der lokalen Anästhesie ergibt. Beim Durchfließen durch die Zahngewebe bewirkt der Gleichstrom jedoch auch deren Polarisation, wodurch der Effekt der Anästhesie verlorengeht. Die Wiederherstellung des erwähnten Effektes erfolgt durch die Depolarisation der Zangewebe, wozu durch sie der Strom in umgekehrter Richtung kurzzeitig durchgeleitet wird. Die Entwicklung der Vorrichtungen zur elektrischen Analgesie, denen zugrunde der Einsatz des elektrischen Stromes liegt, geht den Weg, der auf die Erhöhung des Effektes der Anästhesie gerichtet ist.

Bekannt ist ein Apparat zur elektrischen Anästhesie beim Präparieren von harten Zahngeweben (SU, A, 213987) der in Reihe geschaltet einen Gleichstromregler, einen Gleichstromstabilisator und einen kippschalterförmig ausgebildeten Schalter der Gleichstrompolarität sowie Elektroden zum Anschließen unmittelbar an den Patienten und an ein Mittel zur Einwirkung auf den Patienten enthält, die an den Schalter der Gleichstrompolarität angeschlossen sind. Die eine Elektrode ist als Klipp, der z.B. am Ohrläppchen des Patienten geklemmt wird, und die andere als Klemme ausgebildet, die am Handstück der Bohrmaschine befestigt wird.

Um die elektrische Analgesie der harten Zahngewebe während deren Präparierens durchzuführen, wird der Schalter der Gleichstrompolarität vom Arzt in die Stellung gebracht, in welcher der Klippelektrode das negative Potential und der anderen Elektrode das positive Potential zugeführt wird. Beim Schliessen des elektrischen Kreises der Elektroden über die harten Zahngewebe des Patienten wird der Effekt der Anästhesie beobachtet, der auch von der Polarisation dieser Gewebes begleitet wird. Der Vorgang der Gewebepolarisation beeinträchtigt den Effekt der Anästhesie und ruft Schmerzempfindungen beim Patienten hervor.

Die vorhandene Patientenreaktion auf den eingetretenen Schmerz ist für den Arzt ein Signal, das Zahnpräparieren zu unterbrechen, den Kippschalter umzuschalten und dann, ohne den Zahn zu präparieren, die Elektroden kurzzeitig zu schließen, um die harten Zahngewebe zu depolarisieren. Nach der Depolarisation bringt der Arzt mit dem Kippschalter den Apparat in die Betriebsart elektrische Analgesie und setzt das Präparieren des Zahnes fort.

Aus diesen Ausführungen ergibt sich, daß der Apparat zur lokalen elektrischen Analgesie trotz Durchführung der Depolarisation der harten Zahngewebe die Schmerzempfindungen beim Patienten nicht vollständig verhindert, die durch die Polarisation der festen Zahngewebe hervorgerufen werden, da die Depolarisation im Apparat dadurch erfolgt, daß der Arzt den Kippschalter eben nach der Schmerzempfindung beim Patienten umschaltet.

Bekannt ist auch eine Vorrichtung zur elektrischen Analgesie von Patientengeweben (US,A,3946745), die in Reihe geschaltet einen Generator für unsymmetrische Impulse, an dessen Ausgang die einen Impulse eine größere Breite haben, um die elektrische Analgesie der Patientengewebe zu gewährleisten, die von der Polarisation dieser Gewebe begleitet wird, während die anderen eine geringere Breite zur Gewährleistung der Depolarisation der Patientengewebe haben, einen Stromregler und einen Stromstabilisator enthält, an welchen Elektroden zum Anschließen unmittelbar an den Patienten und an ein Mittel zur Einwirkung auf den Patienten angeschlossen sind.

Bei der beschriebenen Vorrichtung wird der Generator für unsymmetrische Impulse unabhängig davon betrieben, ob das Schließen des elektrischen Kreises der Elektroden über die Patientengewebe vorliegt oder fehlt. Der erste Impuls vom Generator kann daher beim Schließen des elektrischen Kreises der Elektroden beliebige Polarität aufweisen.

Die beschriebene Vorrichtung wird eingesetzt, wenn Veranlagung zum Gebrauch von Rauschmitteln, Asthma, Schlaflosigkeit und Gesichtslähmung behandelt werden. Die Vorrichtung ist darüber hinaus auch zur Anästhesie anwendbar. Ihre Anwendung in der therapeutischen Stomatologie beim Präparieren von harten Zahngeweben, welches von öfteren kurzzeitigen Unterbrechungen des elektrischen Kontaktes der Bohrmaschine mit dem Zahn begleitet wird, ist jedoch wenig effektiv, da es sich ergeben kann, daß auf die harten Zahngewebe nur die Impulse mit der größeren Breite einwirken, die deren elektrische Analgesie gewährleisten, während die Wirkung der Impulse der geringeren Breite, die deren Depolarisation gewährleisten, zeitlich mit dem Verlust des elektrischen Kontaktes der Bohrmaschine mit dem Zahn zusammenfällt. Mit zunehmendem Impuls-Pause-Verhältnis der unsymmetrischen Impulse wird die Möglichkeit erhöht, daß der Impuls mit der geringeren Breite mit dem Verlust des elektrischen Kontaktes zusammenfällt.

Das Ausgeführte ergibt, daß die Vorrichtung zur elektrischen Analgesie von Patientengeweben zwar Impulse zur Gewährleistung der Depolarisation des Patientengewebes erzeugt, ihre Einwirkung auf die harten Zahngewebe des Patienten jedoch nicht garantiert, wodurch der Patient den durch die Polarisation hervorgerufenen Schmerz empfinden kann.

### Offenbarung der Erfindung

Der Erfindung liegt die Aufgabe zugrunde, eine Vorrichtung zur elektrischen Analgesie von Patientengeweben zu schaffen, bei welcher durch die Durchführung der Depolarisation der Zahngewebe des Patienten periodisch und synchron mit dem Zeitpunkt jedes Schließens des elektrischen Kreises der Elektroden über diese Gewebe verminderte Schmerzempfindungen des Patienten ermöglicht wären, die durch die Polarisation der harten Zahngewebe hervorgerufen werden.

Die gestellte Aufgabe wird dadurch gelöst, daß die Vorrichtung zur elektrischen Analgesie von Patientengeweben, enthaltend in Reihe geschaltet einen Generator für unsymmetrische Impulse, an dessen Ausgang die einen Impulse eine größere Breite haben, um die elektrische Analgesie der Patientengewebe zu gewährleisten, die von der Polarisation dieser Gewebe begleitet wird, während die anderen eine geringere Breite zur Gewährleistung der Depolarisation der Patientengewebe haben, einen Stromregler und einen Stromstabilisator, an welchen Elektroden zum Anschließen unmittelbar an den Patienten und an ein Mittel zur Einwirkung auf den Patienten angeschlossen sind, erfindungsgemäß eine Reihenschaltung aus einer Einheit zur Erzeugung eines Signals beim Schliessen des elektrischen Kreises der Elektroden über die Patientengewebe, wobei der Eingang der Einheit an eine der Elektroden angeschlossen ist, und einer Einheit zum Auslösen des Generators für unsymmetrische Impulse ab Impuls der geringeren Breite enthält, die ausgangsseitig an den Eingang des Generators für unsymmetrische Impulse angeschlossen ist.

Zweckmäßig ist, daß bei Vorrichtung zur elektrischen Analgesie von Patientengeweben die Einheit zur Signalerzeugung beim Schließen des elektrischen Kreises der Elektroden über die Patientengewebe als Komparator ausgebildet ist.

Dadurch daß die Reihenschaltung aus der Einheit zur Erzeugung eines Signals beim Schließen des elektrischen Kreises der Elektroden über die Patientengewebe, bei welcher der Eingang an eine der Elektroden angeschlossen ist, und der Einheit zum Auslösen des Generators für unsymmetrische Impulse ab Impuls der geringeren Breite, die ausgangsseitig an den Eingang des Generators für unsymmetrische Impulse angeschlossen ist, in die Vorrichtung zur elektrischen Analgesie von Patientengeweben aufgenommen ist, wird bei beliebigen Pausen während des Zahnpräparierens, die von dem Verlust des elektrischen Kontaktes der Bohrmaschine mit dem Zahn begleitet werden, gewährleistet, daß auf die harten Zahngewebe des Patienten der Impuls einwirkt, der die Depolarisation sicherstellt und den Effekt der Analgesie wiederherstellt, welcher durch die Polarisation des Gewebes verlorengegangen ist, d.h. es wird die Effektivität der Analgesie erhöht.

- 5 -

Die Ausbildung der Einheit zur Signalerzeugung beim Schließen des elektrischen Kreises der Elektroden über die Patientengewebe als Komparator verringert die Trägheit der Vorrichtung bei Durchführung der Depolarisation, wodurch die Effektivität der Analgesie ebenfalls erhöht wird.

## Kurze Beschreibung der Zeichnungen

Nachfolgend wird die Erfindung durch Beschreibung ihrer konkreten Ausführungsform und beigelegte Zeichnungen näher erläutert, in denen es zeigen:

Fig. 1 ein Blockschaltbild der erfindungsgemäßen Vorrichtung zur elektrischen Analgesie von Patientengeweben;

Fig. 2 eine elektrische Prinzipschaltung der erfindungsgemäßen Vorrichtung zur elektrischen Analgesie von Patientengeweben, wobei ein Komparator als Einheit zur Erzeugung eines Signals beim Schließen des elektrischen Kreises von Elektroden über die Patientengewebe verwendet wird;

Fig. 3 ein Zeitdiagramm für unsymmetrische Stromimpulse beim Schließen des elektrischen Kreises der Elektroden über die Patientengewebe.

## Bevorzugte Ausführungsvariante der Erfindung

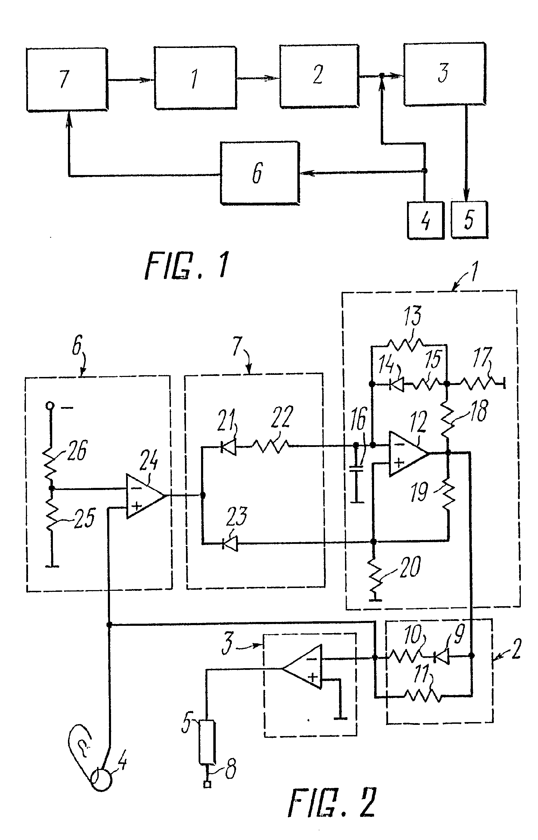

Die Vorrichtung zur elektrischen Analgesie von Patientengeweben enthält einen Generator 1 (Fig.1) für unsymmetrische Impulse, an dessen Ausgang die einen Impulse eine größere Breite haben, um die elektrische Analgesie der Patientengewebe zu gewährleisten, die von der Polarisation dieser Gewebe begleitet wird, während die anderen eine geringere Breite zur Depolarisation der Patientengewebe haben, und dessen Ausgang an den Eingang eines Stromreglers 2 angeschlossen ist, der ausgangsseitig an den Eingang eines Stromstabilisators 3 angeschlossen ist. An den erwähten Eingang bzw. Ausgang des Stromstabilisators 3 ist jeweils eine Elektrode 4, 5 zum Anschließen unmittelbar an den Patienten und an ein Mittel zur Einwirkung auf den Patienten angeschlossen. Die Vorrichtung enthält auch eine Einheit 6 zur Erzeugung eines Signals beim Schließen des elektrischen Kreises der Elektroden über

- 6 -

die Patientengewebe, wobei der Eingang der Einheit an eine der Elektroden, z.B. an die Elektrode 4 angeschlossen ist, und eine eingangsseitig an die Erzeugungseinheit 6 angeschlossene Einheit 7 zum Auslösen des Generators für unsymmetrische Impulse ab Impuls der geringeren Breite, deren Ausgang an den Eingang des Generators 1 für unsymmetrische Impulse angeschlossen ist.

Die erfindungsgemäße Vorrichtung zur elektrischen Analgesie von Patientengeweben ist zur Anwendung beim Präparieren von harten Zahngeweben bestimmt. In diesem Zusammenhang dient als Mittel zur Einwirkung auf den Patienten die Bohrmaschine. Die Elektrode 5 ist dabei als Klemme ausgebildet, die am Handstück 8 (Fig. 2) der Bohrmaschine befestigt wird, während die Elektrode 4 als Klipp ausgebildet ist, der am Ohrläppchen des Patienten befestigt wird.

Bei der hier beschriebenen Ausführungsform der Vorrichtung stellt der Stromstabilisator 3 eine gesteuerte bipolare invertierende Stromquelle mit schwimmender Belastung dar, welche Quelle als Operationsverstärker ausgebildet ist, bei welchem der Ausgang an die ans Handstück 8 der Bohrmaschine anzuschließende Elektrode 5 angeschlossen, der nichtinvertierende Eingang geerdet und die Elektrode 4 an den invertierenden Eingang angeschlossen ist. Der Stromregler 2 ist als Reihenschaltung aus einer Diode 9 und einem Widerstand 10 ausgebildet, parallel zu welchen ein Widerstand 11 angeschlossen ist.

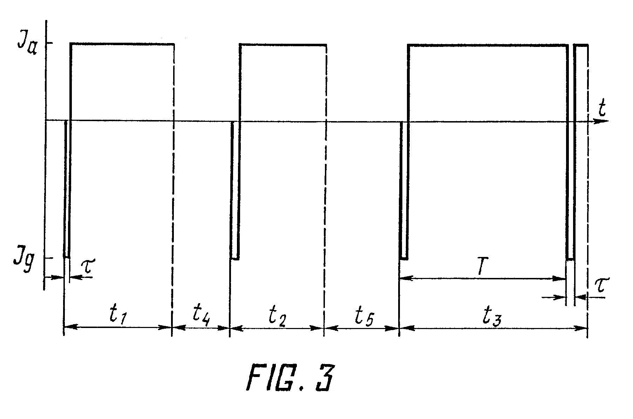

Zum besseren Verständnis der Betriebsweise der Vorrichtung zur elektrischen Analgesie von Patientengeweben zeigt Fig. 3 ein Zeitdiagramm für unsymmetrische Stromimpulse beim Schließen des elektrischen Kreises der Elektroden über die Patientengewebe, in welchem auf der Abszissenachse die Zeit t und auf der Ordinatenachse der Strom $I_a$ zur Gewährleistung der elektrischen Analgesie der Patientengewebe und der Strom $I_g$ zur Gewährleistung der Depolarisation des Patientengewebes abgetragen sind.

Die Widerstandsgröße des Widerstandes 11 (Fig. 2) bedingt den elektrischen Analgesiestrom $I_a$ und die Wider-

standsgröße des Widerstandes 10 (Fig. 2) den Depolarisationsstrom $I_g$ (Fig. 3), der durch die Parallelschaltung der erwähnten Widerstände 10 (Fig. 2), 11 den elektrischen Analgesiestrom $I_a$ überschreitet. Die Diode 9 ist mit dem invertierenden Eingang des erwähnten Operationsverstärkers über den Widerstand 10 verbunden.

Bei dieser Ausführungsform ist der Generator 1 für unsymmetrische Impulse gesteuert, bipolar mit einem Operationsverstärker 12 ausgebildet. Die Unsymmetrie der positiven und negativen Impulse ist dadurch erreicht, daß im Rückkopplungskreis des Operationsverstärkers 12 parallel zum Widerstand 13 die Reihenschaltung aus einer Diode 14 und einem Widerstand 15 geschaltet ist. Die Diode 14 ist katodenseitig an den invertierenden Eingang des Operationsverstärkers 12 angeschlossen. Die Breite der negativen Impulse am Ausgang des Operationsverstärkers 12 wird im wesentlichen durch die Widerstandsgröße des Widerstandes 13 und die Kapazität eines an den invertierenden Eingang des Operationsverstärkers 12 angeschlossenen Kondensators 16 und die der positiven Impulse durch die Durchlaßwiderstandsgröße der Diode 14, die Widerstandsgröße des Widerstandes 15 und die Kapazität des Kondensators 16 bestimmt. Durch verändertes Verhältnis zwischen den Widerstandsgrößen der Widerstände 13 und 15 wird die Breite der unsymmetrischen Impulse des Generators 1 und somit die Dauer der Betriebsarten elektrische Analgesie und Depolarisation eingestellt.

Der Generator 1 für unsymmetrische Impulse enthält auch Widerstände 17, 18, die einen Spannungsteiler bilden, der - wenn die Ausgangsspannung des Generators 1 für unsymmetrische Impulse die der Einheit 7 zum Auslösen des Generators für unsymmetrische Impulse ab Impuls der geringeren Breite überschreitet - parasitäre Generation verhindert, sowie Widerstände 19, 20, die den positiven Rückkopplungskreis des Operationsverstärkers 12 bilden. Dabei sind der Kondensator 16, die Widerstände 17 und 20 geerdet, die Widerstände 18 und 19 an den Ausgang des Operationsverstärkers 12, die Widerstände 20 und 19 an den nichtinvertierenden Eingang des Operationsverstärkers 12 angeschlossen, während die Widerstände 17 und 18 an den gemeinsamen Punkt der Widerstände 13 und 15 angeschlossen sind.

Die Einheit 7 zum Auslösen des Generators für unsymmetrische Impulse ab Impuls der geringeren Breite enthält bei der vorliegenden Ausführungsform eine Diode 21, einen Widerstand 22 und eine Diode 23, die gemeinsam mit der Diode 21 zur Entkopplung der Eingänge des Operationsverstärkers 12 dient. Die Katoden der Dioden 21, 23 sind vereinigt und dienen als Eingang der Einheit 7 zum Auslösen des Generators für unsymmetrische Impulse ab Impuls der geringeren Breite. Die Anode der Diode 23 ist an den nichtinvertierenden Eingang des Operationsverstärkers 12, die Anode der Diode 21 an den Widerstand 22 angeschlossen, der seinerseits an den invertierenden Eingang des Operationsverstärkers 12 angeschlossen ist.

Die Einheit 6 zur Signalerzeugung beim Schließen des elektrischen Kreises über die Patientengewebe ist bei der vorliegenden Ausführungsform als Komparator 24 ausgebildet, der einen Operationsverstärker darstellt. Die Bezugsspannung am invertierenden Eingang des Komparators 24 wird durch den Spannungsteiler aus Widerständen 25, 26 eingestellt. Der Widerstand 25 ist geerdet und der Widerstand 26 an den Minuspol der Speisequelle angeschlossen.

Der Operationsverstärker 12, der Komparator 24 und der Stromstabilisator 3 werden von einer (in der Zeichnung nicht dargestellten) bipolaren Speisequelle versorgt.

Die Vorrichtung zur elektrischen Analgesie von Patientengewebe funktioniert folgenderweise.

Im Zeitpunkt, wo die Elektrode 5 (Fig. 1) mit dem Zahn des Patienten in Berührung kommt, wird der elektrische Kreis der am Handstück 8 (Fig. 2) der Bohrmaschine befestigten Elektrode 5 und der am Ohrläppchen des Patienten befestigten Elektrode 4 geschlossen. Am Eingang des Komparators 24, d.h. am nichtinvertierenden Eingang des Operationsverstärkers, stellt sich eine fast Null gleiche Spannung und an seinem Ausgang eine positive Spannung ein. Die Diode 21 in der Einheit 7 zum Auslösen des Generators für unsymmetrische Impulse ab Impuls der geringeren Breite und die Diode 23 werden geschlossen, während der Kondensator 16 über den Widerstand 13, die Diode 14, den Widerstand 15 und den Widerstand 18 umgeladen und am Ausgang des Operationsverstärkers 12 im Generator 1 für unsymmetrische Impulse ein positiver Kurzzeitimpuls erzeugt wird, der den Kreis aus der Diode 9 und den Widerständen 10, 11 des Stromreglers 2 durchläuft und am Ausgangs des als Stromstabilisator 3 auftretenden Operationsverstärkers einen negativen Impuls der geringeren Breite erzeugt, der die Depolarisation der Patientengewebe, d.h. der harten Zahngewebes gewährleistet. Die Breite $\tau$ (Fig. 3) dieser Impulse und die Stromgröße $j_g$ bewirken die Depolarisation, die im Zeitpunkt jedes Schließes des Kreises der Elektroden 4 (Fig. 2), 5 über die Patientengewebe einsetzt. Die Breite $\tau$ wird gleich 5 bis 150 ms und die des Stromgröße $I_g$ gleich 80 bis 150 $\mu$A eingesetzt.

Nach der Betriebsart Depolarisation wird die Betriebsart Elektroanalgesie eingestellt, die durch die Impulse der grösseren Breite gewährleistet wird, deren Stromgröße $I_a$ (Fig. 3) nur durch die Widerstandsgröße des Widerstandes 11 (Fig. 2) im Stromregler 2 und die Breite durch die Kapazität des Kondensators 16 und die Widerstandsgröße der Widerstände 13, 18, 19, 20 im Generator 1 bedingt wird. Die Breite dieser Impulse wird gleich 3 bis 5 s und die Stromgröße $I_a$ gleich 30 bis 100 $\mu$A eingestellt.

Unterschreitet die Zeit $t_1$ (Fig. 3), $t_2$ für das Zahnpräparieren die Schwingungsperiode T des Generators 1 (Fig. 2) für unsymmetrische Impulse, wird die Betriebsart Depolarisation wiederholt nicht eingestellt. Überschreitet die Zeit $t_3$ (Fig. 3) für das Zahnpräparieren die erwähnte Periode T, wird die Betriebsart Depolarisation bereits automatisch wiederholt eingestellt. Bei beliebigen Unterbrechungen während des Zahnpräparierens, die in Fig. 3 als Intervalle $t_4$, $t_5$ dargestellt sind, wird gewährleistet, daß auf die harten Zahngewebe mit Depolarisationsimpulsen eingewirkt wird, wodurch die Schmerzempfindun-

- 10 -

gen beim Patienten vermindert, die durch die Polarisation der harten Zahngewebe hervorgerufen wird, und folglich die Wirksamkeit der Anästhesie erhöht wird.

Die erfindungsgemäße Vorrichtung zur elektrischen Analgesie von Patientengeweben wurde in Kliniken an Menschen bei der therapeutischen Stomatologie erprobt, wobei unkomplizierte Caries superficialis, media und profunda, besonders am Zahnhals behandelt, Pulpakammer eröffnet und Kronenpulpa bei akuter und chronischer Pulpitis amputiert wird. Bei allen Patienten betrug der Strom zur Gewährleistung der elektrischen Analgesie 60 $\mu$A, der Strom zur Gewährleistung der Depolarisation 100 $\mu$A, die Impulsbreite des Stromes zur Gewährleistung der elektrischen Analgesie 5 s, die Impulsbreite des Stromes zur Gewährleistung der Depolarisation 50 ms.

Die Versuchsgruppe bestand aus 100 Patienten (Männer und Frauen) im Alter von 10 bis 59 Jahre. Die Wirksamkeit der Anästhesie wurde durch Abfragen nach dem erfolgten Präparieren der harten Zahngewebe erfaßt. Bei 40 Patienten kam es während des Präparierens der harten Zahngewebe zur periodischen Abschaltung der Vorrichtung, wobei 38 Patienten Schmerzempfindungen hatten. Es wurde beobachtet, daß bei Caries profunda und am Zahnhals vollständige und teilweise Anästhesie 95 % und bei Caries superficialis 88 % der Fälle betrug. Bei 5 Patienten im Alter von 41 bis 57 Jahre war keine Anästhesie festzustellen. Es wurde auch beobachtet, daß bei älteren Patienten die vollständige Anästhesie viel seltener als bei Personen mittleren und besonders jungen Alters eintrat. Es kam auch ein Fall vor, wo der Effekt der Anästhesie vorlag und fehlte, als beim gleichen Patienten verschiedene Zähne präpariert wurden.

Bei Eröffnung der Pulpakammer und Amputierung der Kronenpulpa bei akuter und chronischer Pulpitis betrug vollständige und teilweise Anästhesie 91 % der Fälle.

Zu beobachten war, daß die Vorrichtung keine Nebenwirkungen und keine Kontraindikationen hat.

Industrielle Anwendbarkeit

Die Erfindung ist erfolgreich in therapeutischen Stomatolagie anwendbar, wenn harte Zahngewebe zur Behandlung von unkomplizierter Caries superficialis, media und profonda, besonders am Zahnhals, präpariert werden sowie Pulpakammer eröffnet und Kronenpulpa bei akuter und chronischer Pulpitis amputiert wird. Besonders effektiv ist die Erfindung in der Kinderstomatologie anwendbar.

PATENTANSPRÜCHE

1. Vorrichtung zur elektrischen Analgesie von Patientengeweben, enthaltend in Reihe geschaltet einen Generator (1) für unsymmetrische Impulse, an dessen Ausgang die einen Impulse eine größere Breite haben, um die elektrische Analgesie der Patientengewebe zu gewährleisten, die von der Polarisation dieser Gewebe begleitet wird, während die anderen eine geringere Breite zur Gewährleistung der Depolarisation der Patientengewebe haben, einen Stromregler (2) und einen Stromstabilisator (3), an welchen Elektroden (4,5) zum Anschließen unmittelbar an den Patienten und an ein Mittel zur Einwirkung auf den Patienten angeschlossen sind, dadurch g e k e n n z e i c h n e t , daß sie eine Reihenschaltung aus einer Einheit (6) zur Erzeugung eines Signals beim Schließen des elektrischen Kreises der Elektroden über die Patientengewebe, wobei der Eingang der Einheit an eine der Elektroden (4, 5) angeschlossen ist, und einer Einheit (7) zum Auslösen des Generators für unsymmetrische Impulse ab Impuls der geringeren Breite enthält, die ausgangsseitig an den Eingang des Generators (1) für unsymmetrische Impulse angeschlossen ist.

2. Vorrichtung zur elektrischen Analgesie nach Anspruch 1, dadurch g e k e n n z e i c h n e t , daß die Einheit (6) zur Signalerzeugung beim Schliessen des elektrischen Kreises der Elektroden über die Patientengewebe als Komparator (24) ausgebildet ist.

FIG. 1

FIG. 2

FIG. 3

## I. CLASSIFICATION OF SUBJECT MATTER (if several classification symbols apply, indicate all) 6

According to International Patent Classification (IPC) or to both National Classification and IPC

IPC⁴ A 61 N 1/34, 1/08, // A 61 C 19/08

## II. FIELDS SEARCHED

| Minimum Documentation Searched 7 | |
|---|---|
| Classification System | Classification Symbols |
| IPC⁴ | A 61 N 1/34, 1/08, 1/36, A 61 C 19/08 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | US, A, 4088141, (Stimulation Technology, Inc.), 9 May 1978 (09.05.78), see column 3, lines 31-33, 42-43, fig. 1 | 2 |
| A | US, A, 3946745, (Biopulse Company Limited), 30 March 1976 (30.03.76), see column 3, lines 61-67, column 4, lines 1-23, fig. 2 | 1 |
| A | DE, A1, 3047402, (Preywisch, Wolfgang), 22 July 1982 (22.07.82), see fig. 6, the claims | 1 |

---

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 5 May 1988 (05.05.88) | 25 May 1988 (25.05.88) |
| International Searching Authority ISA/SU | Signature of Authorized Officer |